# NEUE EUROPÄISCHE PATENTSCHRIFT

(11) **EP 0 103 196 B2**
(45) Veröffentlichungstag und Bekanntmachung des Hinweises auf die Entscheidung über den Einspruch: **12.10.1994**
(45) Hinweis auf die Patenterteilung: 25.10.1989
(21) Anmeldenummer: 83108068.4
(22) Anmeldetag: 16.08.1983
(51) Int. Cl.: A61L 2/04, A61K 37/02

(54) **Pasteurisiertes Human-Fibrinogen (HF), Verfahren zu dessen Herstellung und dessen Verwendung**
Pasteurized human fibrinogen, method of preparing it and its use
Fibrinogène humain pasteurisé, procédé de préparation et utilisation

(30) Priorität: 19.08.1982 DE 3230849
(43) Veröffentlichungstag der Anmeldung: 21.03.1984
(73) Patentinhaber: BEHRINGWERKE Aktiengesellschaft, 35001 Marburg (DE)
(72) Erfinder: Kumpe, Gerhardt, D-3552 Wetter (DE); Wormsbächer, Wilfried, D-3575 Kirchhain (DE); Heimburger, Norbert, Prof. Dr., D-3550 Marburg 1 (DE); Fuhge, Peter, Dr., D-3551 Lahntal-Caldern (DE); Preis, Hans Martin, D-3550 Marburg 16 (DE)

(56) Entgegenhaltungen:
- EP-A- 0 018 561
- EP-A- 0 035 204
- EP-A- 0 053 338

## Beschreibung

Die Erfindung betrifft ein pasteurisiertes Human-Fibrinogen (HF), Verfahren zu dessen Herstellung und dessen Verwendung in Arzneimitteln.

HF ist ein sehr wichtiger Blutgerinnungsfaktor, der am Ende der sogenannten Gerinnungskaskade steht: HF wird bei Aktivierung des Gerinnungssystems, beispielsweise nach Verletzungen, durch Thrombin von seiner löslichen Form in das unlösliche Fibrin umgewandelt, das wesentlich zur Hämostase und Wundheilung beiträgt. Das HF ist der Gerinnungsfaktor, der als einzig wirkliches Substrat von allen anderen Gerinnungsfaktoren und auch in der höchsten Konzentration im Plasma vorkommt, nämlich zwischen 250 und 400 mg %. Wegen seiner Bedeutung für die Blutstillung und Wundheilung wird HF klinisch angewendet, z. B. bei Verbrauchsreaktionen wie der disseminierten intravasculären Coagulation (DIC) bei Septicämien. Daneben wird das Fibrinogen neuerdings auch als sog. "Kleber" anstelle von Nähten bzw. zur Abdichtung von Nähten verwendet, vorwiegend bei chirurgischen Eingriffen und speziell an Wveichteilorganen wie Leber und Milz.

Das aus Plasma von Hepatitis-B-Trägern gewonnene HF birgt das Risiko einer Hepatitisübertragung in sich, da es aus Plasmafraktionen gewonnen wird, in denen es vergesellschaftet mit anderen hochnolekularen und schwerlöslichen Proteinen vorkommt, die als sog. Hepatitis-Fänger -Fraktionen betrachtet werden. Aus besagten Gründen wurde HF bislang nur bei ausgesprochen vitalen Indikationen verwendet, weil sich nämlich das Risiko einer Hepatitisübertragung nicht mit absoluter Sicherheit ausschalten ließ. Dazu trug auch bei, daß es bisher weder für die Hepatitis B und schon gar nicht für die Nicht-A/Nicht-B-Hepatitis einen absolut verläßlichen Test gibt.

Aus dieser Situation resultierte der Bedarf an einem hepatitissicheren HF, wie es sich z. B. durch die Kombination eines Fraktionierungsverfahrens mit einer nachfolgenden Pasteurisierung herstellen läßt. Nun ist jedoch jedem Fachmann auf dem Gebiet der Blutgerinnung bekannt, daß man Plasma durch Erhitzen auf 58°C (3 min) defibrinieren kann, weil nämlich das HF zu den hitzelabilen Proteinen gehört. So kommt man bei der Erhitzung des F VIII, wie sie in der DE-P-2 916 711 "Blutgerinnungsfaktoren und Verfahren zu ihrer Herstellung" (US-P 4 297 344) beschrieben wird, zu einem fibrinogenfreien Präparat. In Übereinstimmung damit sind auch die Befunde in der EP-0 035 204, in der auf Seite 23/24 festgestellt wird, daß HF nur in einer Konzentration von 0,05 - 0,4 % durch bekannte Stabilisatoren wie Kohlenhydrate vor thermischer Inaktivierung geschützt werden kann.

Aus der Veröffentlichtung von Ly und Godal in Haemostasis 1: 204-209 (1972/73) ist bekannt, daß geringe Mengen von Calcium (Konzentration 1 mMol/Liter) die Denaturierung von Fibrinogen in Lösung (Konzentration: 3,3 mg/ml) bei einer Erhitzung auf 47 _{°} C verhindern können, während höhere Konzentrationen von Calcium Fibrinogen ausfällen. Eine Erhitzung auf 47 _{°} C reicht jedoch für eine Pasteurisierung, das heißt eine sichere Abtötung von allen vermehrbaren Keimen nicht aus.

Nach dem erfindungsgemäßen Verfahren wird HF in einer Reinheit von 85 % in einer Konzentration von 1 bis 7 % in einer wässrigen Lösung, die mindestens 1 g Atom Calcium/Mol HF enthält, von pH 6 - 8 aufgenommen und als Stabilisatoren Kohlenhydrate (Mono- oder Oligosaccharide oder Zukkeralkohole) oder eine Mischung solcher Kohlenhydrate und Aminosäuren, vorzugsweise Saccharose und Glycin, zugegeben. Calcium, die Kohlenhydrate und Aminosäuren werden in einer für die Stabilisierung ausreichenden Konzentration zugegeben und in der zweckmäßigen Reihenfolge Ca²⁺, Kohlenhydrate, z. B. Saccharose, und Aminosäure, z. B. Glycin, der HF-Lösung zugesetzt. Solche Lösungen lassen sich über mehrere Stunden bei Temperaturen bis zu 60 _{°} C halten, pasteurisieren.

Gegenstand der Erfindung ist somit ein Pasteurisierungsverfahren für HF in wässriger Lösung, dadurch gekennzeichnet, daß es neben üblichen Stabilisatoren in Gegenwart von Calonen in einer Konzentration von 1 bis 37,8 . 103 g Atome, vorzugsweise 1 bis 10 g Atome pro Mol Humanfibrinogen durchgeführt wird.

Von den üblichen Stabilisatoren wird Saccharose in einer Konzentration von 35 bis 60 g/100 ml Lösung und Glycin in einer Konzentration von 0,5 bis 3 Mol/I bevorzugt.

Diese Lösung wird in an sich üblicher Weise erhitzt. Zweckmäßig ist die Erhitzung auf mindesten 60°C und höchstens 100°C für mindestens 10 Stunden und höchstens 24 Stunden.

Die in den gereinigten HF-Lösungen ggfs. vorhandenen Fibrin-Polymere, deren Konzentration während der Erhitzung auf 60°C noch zunehmen kann, werden nach Erhitzen ggfs. durch eine Fällung, vorzugsweise mit 0,25 bis 1,5 Mol/I Glycin abgetrennt und das HF aus dem Überstand durch Erhöhung der Konzentration des Fällungsmittels, z. B. der Glycin-Konzentration, auf mindestens 2,2 Mol/I (2,0 - 2,7 Mol/I) gewonnen und gleichzeitig von den Stabilisatoren getrennt.

Mit diesen Schritten kommt man zu einem sehr sauberen, nativen polymerfreien, pasteurisierten HF mit guten Löslichkeits-Eigenschaften, das sich ganz breit therapeutisch verwenden läßt, vorzugsweise als Infusionslösung mit ca. 2 % (w/v) HF oder als Gewebekleber mit ca. 10% (w/v) HF. Solche Mittel sind ebenfalls Gegenstand der Erfindung.

Die Erfindung wird am nachstehenden Beispiel näher er läutert:

### Beispiel:

### 1. Ausgangsmaterial

500 g fibrinogenhaltiger Rückstand einer 2,7 Mol/I Glycin-Fällung, wie sie bei der Herstellung von Faktor VIII-Konzentrat HS (DE-P-2 916 711) anfällt, wurde unter Erwärmen auf 37 °C und Rühren in 1250 ml 0,15 Mol/I NaCI-Lösung gelöst und mit 2 N NaOH auf pH 7,5 eingestellt. Nach Lösen wurden 1700 ml einer 6 %igen Fibrinogen-Lösung erhalten.

### 2. Stabilisierung und Pasteurisierung

A) 1700 ml Fibrinogen-Lösung aus I. wurden mit 5 m Mol/I CaC1₂ x 2 H₂0 versetzt (1250 mg) und bis zur Lösung gerührt. Nachdem das zugesetzte CaC1₂ voll ständig gelöst war, wurden unter Rühren und Erwärmen 60 % (w/v) Saccha- rose (1700 g) zugefügt.
   Nachdem sich die Saccharose vollständig gelöst hatte, wurden 1 Mol/I Glycin (127,5 g) zugegeben. Nach vollständigem Lösen des Glycins wurde der pH-Wert mit 2 N NaOH auf pH 7,5 eingestellt. Es resultierte eine opaleszente, sehr viskose Lösung, die anschließend 10 Std. bei 60 °C in einem Wasserbad inkubiert wurde.
   Die erhitzte Fibrinogen-Lösung wurde zur Abtrennung von Fibrinpolymeren (3) und der Isolierung des Fibrinogens (4) wie folgt weiterbehandelt:
   2,9 I stabilisierte, erhitzte Fibrinogen-Lösung wurden nach Temperierung auf 20 °C mit 8,7 I Puffer (0,06 mol/I NaCI - 0,02 Mol/I Tri-Na-Citrat) im Verhältnis 1 : 4 verdünnt und 11,6 I erhitzte verdünnte Fibrinogen-Lösung erhalten, die mit Glycin fraktioniert gefällt wurde.
B) Das vorstehende Verfahren A) läßt sich auch mit einer vergleichsweise hohen Konzentration von Ca-Ionen, z. B. mit 1,8 Mol/I CaCl₂ x 2 H₂0 (449,8 g) mit dem gewünschten Stabilisierungseffekt durchführen.

### 3. Abtrennung der hochmolekularen Bestandteile, vornehmlich der Fibrin-Polymere

Zu der erhitzten und anschließend verdünnten Fibrinogen-Lösung wurden bei 37°C 86,25 g Glycin/I (= 1,15 Mol/I) zugefügt. Nach Lösen wurde auf 20 °C abgekühlt und die erhaltene Fällung in der Stock-Zentrifuge abgetrennt.

### 4. Isolierung des Fibrinogens aus dem Stabilisatormedium

Der erneut auf 37 °C erwärmte 1,15 Mol/I Glycinhaltige Überstand aus 3. wurde mit weiterem Glycin auf eine Endkonzentration von 2,2 Mol/I gebracht und so lange gerührt (30 min), bis das Glycin vollständig gelöst war.

Die Fällung wurde auf 20 °C abgekühlt und in der Stock-Zentrifuge abgetrennt.

Für die Verwendung als Humanfibrinogen für die intravenöse Infusion wurde der Fibrinogen-Rückstand 2 %ig in 2500 ml 0,01 Mol/I Tri-Na-Citrat - 0,15 Mol/I Nacl gelöst, gegen den gleichen Puffer dialysiert und nach Klär- und Sterilfiltration in 60 ml Volumina (1 g HF) abgefüllt und lyophilisiert.

Für die Herstellung von "Fibrinkleber" wurde ein nach dem gleichen Verfahren gewonnener Fibrinogen-Rückstand 4 %-ig in 1250 ml folgender Salzlösung aufgenommen: 0,05 Mol/I NaCI; 0,005; Mol/I Tri-Na-Citrat; 0,01 Mol/I NaHC0₃; 0,33 % L-Arginin monohydrochlorid, pH 7,5; dialysiert (Lösepuffer) und anschließend ultrazentrifugiert: 1 Stunde 35.000 x g. Nach Klär- und Sterilfiltration erfolgt die Lyophilisation in 4 ml Abfüllungen.

Dieses Produkt ist nach Rekonstitution und in Verbindung mit F XIII und Thrombin für die Klebung von Weichteilen und die Abdichtung von Gefäßnähten geeignet.

## Patentansprüche

1. Verfahren zur Pasteurisierung von Human-Fibrinogen in wässriger Lösung, dadurch gekennzeichnet, daß das Human-Fibrinogen in wässriger Lösung neben üblichen Stabilisatoren Calcium in einer Konzentration von 1 bis 37,8 . 103 g Atome/Mol Human-Fibrinogen enthält und auf eine Temperatur von 60 bis 100°C erhitzt wird.

2. Verfahren nach Anspruch 1, dadurch gekennzeichnet, daß die üblichen Stabilisatoren Mono- oder Oligosaccharide oder Zuckeralkohole und eine Aminosäure sind.

3. Verfahren nach Anspruch 2, dadurch gekennzeichnet, daß als Oligosaccharid Saccharose in einer Konzentration von 35 bis 60 g/100 ml Lösung verwendet wird.

4. Verfahren nach Anspruch 2, dadurch gekennzeichnet, daß als Aminosäure Glycin in einer Konzentration von 0,5 bis 3 mol/I verwendet wird.

5. Verwendung, eines gemäß dem Verfahren nach mindesten einem der Ansprüche 1 - 4 erhalten pasteurisierten Human-Fibrinogens zur Herstellung einer Infusionslösung.

6. Verwendung eines gemäß dem Verfahren nach mindesten einem der Ansprüche 1 - 4 erhalten pasteurisierten Human-Fibrinogens zur Herstellung eines Gewebeklebers.

## Claims

1. A process for the pasteurization of human fibrinogen in aqueous solution, wherein the human fibrinogen contains calcium in a concentration of 1 to 37.8 x 10³ g atoms/mol of human fibrinogen in aqueous solution, together with customary stabilizers, and is heated at a temperature of from 60 to 100 _{°} C.

2. The process as claimed in claim 1, wherein the customary stabilizers are monosaccharides or oligosaccharides or sugar alcohols and an amino acid.

3. The process as claimed in claim 2, wherein sucrose is used as the oligosaccharide in a concentration of 35 to 60 g/100 ml of solution.

4. The process as claimed in claim 2, wherein glycine is used as the amino acid in a concentration of 0.5 to 3 mol/I.

5. The use of a pasteurized human fibrinogen obtained according to the process as claimed in at least one of claims 1-4 for producing an infusion solution.

6. The use of a pasteurized human fibrinogen obtained according to the process as claimed in at least one of claims 1-4 for producing a tissue adhesive.

## Revendications

1. Procédé pour la pasteurisation de fibrinogène humain en solution aqueuse, caractérisé en ce que la solution aqueuse de fibrinogène humain contient au côté des stabilisateurs conventionnels, du calcium en une concentration variant de 1 à 37,8.10³ g atome/mol de fibrinogène humain et en ce qu'elle est chauffée à une température de 60 à 100°C.

2. Procédé selon la revendication 1, caractérisé en ce que les stabilisateurs conventionnels sont des mono- ou oligosaccharide ou bien glucoside, et un acide aminé.

3. Procédé selon la revendication 2, caractérisé en ce que l'on utilise en tant qu'oligosaccharide du saccha-rose à une concentration allant de 35 à 60 g/100 ml de solution.

4. Procédé selon la revendication 2, caractérisé en ce que l'on utilise en tant qu'acide aminé de la glycine à une concentration variant de 0,5 à 3 mol/I.

5. Utilisation de fibrinogène humain pasteurisé selon l'un des procédés d'au moins l'une des revendications 1 à 4, pour la préparation d'une solution pour perfusion.

6. Utilisation de fibrinogène humain pasteurisé obtenu selon le procédé d'au moins l'une des revendications 1 à 4 pour la préparation d'une colle pour tissus.
